# EUROPEAN PATENT SPECIFICATION

(11) **EP 1 904 117 B1**
(45) Date of publication and mention of the grant of the patent: **21.09.2011**
(21) Application number: 06748036.8
(22) Date of filing: 14.06.2006
(51) Int. Cl.: A61L 9/20

(54) **METHOD FOR TREATING CONTAMINATED AIR WITH UV-LIGHT**
VERFAHREN ZUR BEHANDLUNG VON KONTAMINIERTER LUFT MIT UV-LICHT
METHODE DE TRAITEMENT D'AIR CONTAMINE PAR UN RAYONNEMENT UV

(30) Priority: 07.07.2005 SE 0501593
(43) Date of publication of application: 02.04.2008
(73) Proprietor: Lejondahl, Lars-Erik, 641 34 Katrineholm (SE)
(72) Inventor: Lejondahl, Lars-Erik, 641 34 Katrineholm (SE)
(74) Representative: Holmberg, Magnus
(86) International application number: PCT/SE2006/050196
(87) International publication number: WO 2007/008164

(56) References cited:
- WO-A1-03/061717
- US-A- 5 968 455
- US-A1- 2004 013 583
- US-A1- 2004 161 371

## Description

### Technical field

The present invention relates to a system that includes a suction device for the extraction of contaminated air, in which an air stream is irradiated with ultraviolet light, UV-light, emitted from UV-lamps which are adapted to treat said air such as to render the contaminants innocuous. A method associated with the system relates to the treatment of the contaminated air stream, optionally with ozone, wherein the contaminated stream of air is irradiated with UV-light emitted from UV-lamps.

### Background of the invention

Methods, systems, and devices for rendering air-carried contaminants innocuous with the aid of UV-light are known to the art. Such systems, etc., are described *inter alia* in international patent publication WO03/61717. The method described in this international patent publication is based on the irradiation of a contaminated air stream with UV-light emanating from UV-lamps whose radiation is regulated in different ways so as to obtain desired ozone formation. The method is carried out with the aid of regulating means that enable the air stream to be exposed to UV-light over periods of different durations, and with the aid of sensors which indicate the presence of contaminants and ozone respectively in, *inter alia,* the cleansed exhaust air exiting from the system. Values relating to air speed, the duration of irradiation of the air with ultra violet light, and the intensity of the ultra violet radiation are set with the aid of a microcomputer. However, the radiation emitted from the UV-lamps decreases with time and the lamps are dirtied successively, therewith impairing the result of the treatment. The UV-lamps must therefore be cleaned and replaced at certain time intervals in order to enable the air contaminants to be kept at acceptable levels. Neither shall the UV-radiation be so strong as to result in an overproduction of the ozone. Obnoxiously smelling contaminants present in air channels, flues and the like, such as grease or fat deposits, also increase the risk of fire and require cleaning of such channels.

One drawback with the known system, however, is that the sensors are easily dirtied and are liable to emit misleading signals that can result in an excessively high concentration of contaminants in the exhaust air leaving the system. Moreover, some contaminants can not be detected with suitable commercially available sensors.

### Summary of the invention

The contaminated air stream is irradiated in the system with UV-light emitted from UV-lamps, which may be lamps that emit UV-light with a wavelength of about 254 nm that kills bacteria in the contaminated air, or lamps that emit UV-light with a wavelength of about 185 nm which splits organic molecules and converts oxygen to ozone, which then oxidizes the split molecules. The following description does not differentiate between these two types of lamps.

The UV-lamps have a given length of life which is determined by virtue of causing the set UV-radiation emitted by the lamps to decrease in accordance with a first function from an initial state when activating the system to a lowest acceptable theoretical value. The UV-lamps shall be cleaned at a time interval which is determined by the fact that the radiation actually emitted has been decreased to a smallest desired value. Cleaning of the lamps is effected at time intervals calculated with the aid of
- the value of the UV-radiation in the initial state;
- the value of the smallest desired actual UV-radiation;
- the first function for reduction of the UV-radiation during a system operating period; and
- a second function for the reduction of UV-radiation due to dirtying of the UV-lamps.

The functions of the UV-radiation may either be linear or have other forms, for instance due to variations in dirtying of the lamps over shorter or longer periods of time. The functions may also change appearance with time as the actual data is logged-in. It is also possible to choose between two or more function variants, particularly between variants of the second UV-light function, which is governed by external factors. The second UV-function, which relates to dirtying of the UV-lamps, may have different appearances due to variations in a working area. For example, in the case of garbage collections contamination of the ambient air is greater during the summer months, when the temperature is higher than during the winter months. The cleaning intervals are therefore shorter during the summer months. This is taken into account by structuring the computing unit in the system to choose other functions that are dependant on the time of year, this choice being made manually or in some other ways. In the case of restaurant kitchens and commercial kitchens the variations that occur during the day are more noticeable although being stable over the year, meaning that it may be necessary to change the UV-light functions instead in accordance with a different operating conditions. The UV-light function for the technical UV-radiation of the UV-lamps is adjusted to a new first function by measuring the UV-radiation after cleaning the UV-lamps and comparing this radiation with an earlier set value at this point in time. The second UV-light function is adjusted to a new second function by selectively or subsequent to said set time interval measuring the UV-radiation from the dirtied UV-lamps and comparing this measured radiation with an earlier set value for this point in time from the earlier set second function, wherein the set value of the cleaning interval may be either increased or decreased.

If cleaning of the UV-lamps is not undertaken at a pre-determined time point, but at an earlier or a later time point the time point for the next cleaning operation is calculated on the basis of the actual cleaning time point. Attention is paid to both the first and the second UV-light function in this regard. Further characteristic features and details of the present invention will be apparent from the accompanying drawings and from the accompanying claims.

### Brief description of the drawings

The invention will now be described with reference to an exemplifying embodiment and also with reference to the accompanying drawings.

The invention proposes the use of at least UV-lamps that emit wavelengths of 185 and 254 nm and which are ozone productive.
Figure 1 illustrates schematically the radiation from UV-lamps as a function of time and with an inputted time interval for cleaning of the UV-lamps;
Figure 2 illustrates schematically an example of the variation of the contamination intensity over a period of a calendar day;
Figure 3 illustrates schematically the radiation from UV-lamps as a function of time, including difference time intervals for cleaning the UV-lamps; and
Figure 4 illustrates schematically the radiation from UV-lamps as a function of time, and shows reduction of the radiation from the UV-lamps down to a desired value.

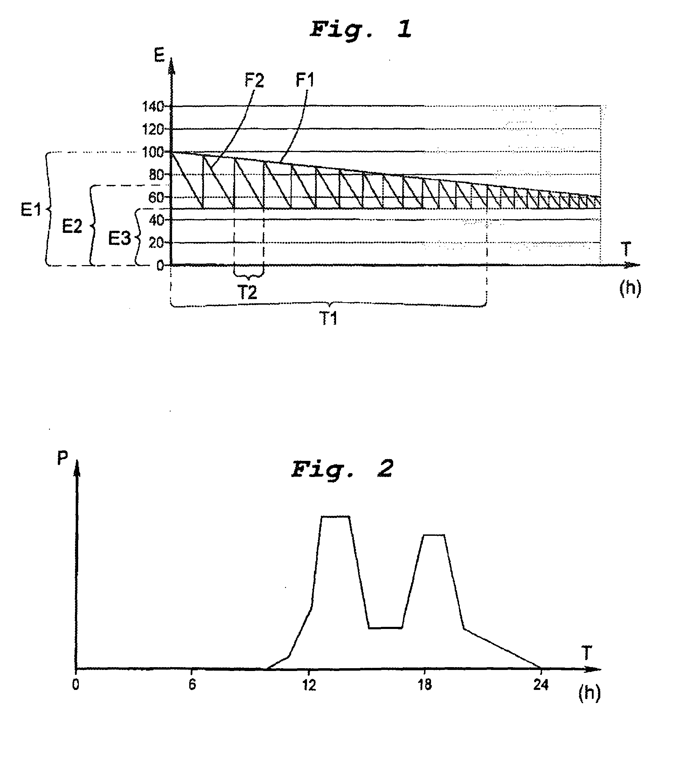

The system in which the inventive method can be applied includes a suction device for the treatment of air that is contaminated with organic substances, with the aid of UV-lamps such as to render the contaminants innocuous. The method illustrated in figure 1 is concerned with controlling the treatment of the contaminated air stream with UV-light, wherewith the contaminated air stream is irradiated with UV-light from UV-lamps, this light being effective to exterminate bacteria and/or split organic molecules, and also in converting oxygen into ozone which then oxidizes the split molecules. The UV-lamps have a given length of life T1, which is determined by the reduction of the set emitted UV-radiation E in accordance with a first function F1 from an initial state when the system is first activated E1 to a smallest theoretically acceptable value E2, wherewith the UV-lamps shall be cleaned at a time interval T2 which is determined by the reduction of the radiation actually emitted to a smallest desired value E3, which may also be comprised of a third function F3. The time interval T2 in which the UV-lamps shall be cleaned is calculated with the aid of
- the value of the UV-radiation at the initial starting state E1,
- the value of the smallest desired actual UV-radiation E3;
- the first function F1 for reducing the UV-radiation during the time period T; and
- a second function F2 for reducing the UV-radiation from each cleaning occasion.
The first function F1 set in the initial starting state and the second function F2 and associated time intervals T2 can be changed successively by observing the result of the treatment on the air stream and the soiling of UV-lamps and/or air channels.

Observations can be made, for instance, by smelling the treated air so as to determine whether or not the treatment has been successful in removing smell, or whether the UV-radiation is excessively intensive and results in an excessively strong ozone smell. Dirtying of UV-lamps, surrounding surfaces and air channels can be determined visually or measured with the aid of appropriate measuring apparatus. The third function F3 and associated smallest desired level E3 of UV-radiation can also be changed in this connection.

The first function F1 in respect of the time-set UV-radiation from the UV-lamps normally decreases linearly whereas the second function F2 can vary greatly as time passes, due to dirtying of the lamps and because of the intensity of the contaminating source and because of the nature of the activity that generates the contaminated air. The second function F2 may need to be changed also with respect to variations in dirtying of the lamp during the day. In the case of a deep-fryer used in a restaurant, dirtying of the UV-lamps will occur generally stepwise as shown in figure 2, where the intensity P to which the lamps are dirtied is highest at lunchtime and in the evening whilst being essentially zero at other times. On the other hand, in the case of a continuous process contaminated air is treated with the same intensity during all times of the day, resulting in uniform dirtying of the UV-lamps.

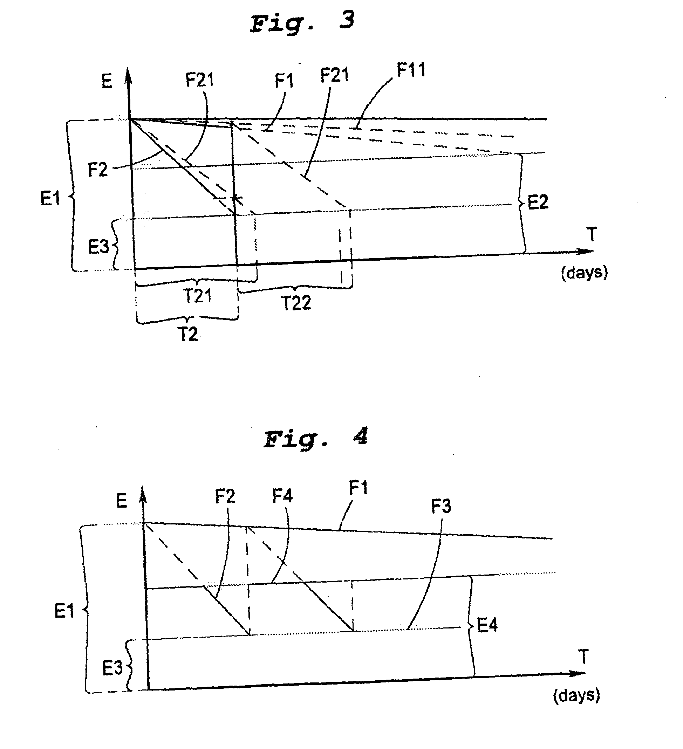

A contaminated air purifying system described above is installed on the basis of functions and on a cleaning interval schedule which has been reached by experience and which may have an appearance similar to that shown in figure 1. The first function F1 for the technical UV-radiation of the UV-lamps and the second function F2 for the UV-radiation as the lamps are dirtied are adjusted either as a result of system observations or as a result of first measuring the UV-radiation from the dirtied UV-lamps, for instance on a lamp cleaning occasion, and of measuring the radiation subsequent to cleaning the lamps. The result of these measurements can be presented in the manner shown in figure 3. After the set time interval T2 for cleaning the UV-lamps it may be found that dirtying of the lamps was less then was expected, thereby indicating that the time interval was too short and should be substituted for a longer time interval T21, meaning that the second function F2 should be changed in a manner corresponding to F21, as shown in figure 3.

When measuring the UV-radiation after cleaning the UV-lamps the first function F1 for the technical UV-radiation of the lamps, as shown in figure 3, may be found to be erroneous and result in a higher radiation level than the radiation level first set, as in the case of the example.

This results in a new first function F11, and it is necessary to start from this new first function when determining the time interval for the next cleaning operation T22 with the aid of said new second function F21. The same procedure is taken if dirtying of the UV-lamps is found to be greater than first expected or if radiation of the UV-lamps should decrease more rapidly.

Different systems may have different values of UV-radiation in the initial state E1, meaning that it may be necessary to measure the UV-radiation in the initial state so that the first function F1 can be placed at the correct level from the beginning. It may also be necessary to place the second function F2 at the right level, by measuring initially the concentration of contaminants in the air. The measurements may be logged-in over a long period of time and then used to create new functions F1, F2 or to determine alarm threshold values and to achieve automatic adjustment of applied functions.

In the case of particularly sensitive or susceptible applications with which great demands on air purification are involved or where a fire may be liable to occur, for instance in the air channels of, e.g., a restaurant in a shopping mall, different sensors may be used to monitor the system. If the cleaning interval T2 tends to become too short, i.e. shorter than a pre-determined value, the system will generate a signal indicative of a lamp-exchange based on the value logged into the system. A UV-sensor may also be arranged to measure the UV-radiation at closer time intervals than the cleaning intervals T2 inputted in the system, possibly continuously when this is possible in view of the sensor itself being dirtied. It can also be determined with the aid of the UV-sensor if dirtying of the UV-lamps takes place more rapidly than the inputted value of the cleaning interval T2.

The concentration of contaminants that are carried by the air through the system can be measured with the aid of a contamination sensor. If, for instance the cleaning interval T2 is too long, the system will deliver a signal indicating that this interval should be shortened and that also a check shall be made to ascertain that the UV-lamps submit sufficient radiation. Such a contamination sensor may optionally be combined with a UV-sensor. Changes in the cleaning interval T2 may also be carried out automatically in the associated control unit.

The irradiation effect from the UV-lamps may be reduced to a desired level E4 or to a fourth function F4 as shown in figure 4 with the aim of avoiding an overproduction of ozone. This enables the system to be over dimensioned with regard to UV-lamps that are able to generate more UV-radiation than was initially required, which also extends the lamp replacement time. In order to sustain the level E4, it is necessary to adjust the radiation effect with respect to dirtying of the UV-lamps in accordance with the second function F2 and also in accordance with the first function F1. The reduction is thus ceased when dirtying of the UV-lamps has reached UV-light function F2, when the system functions in the above mentioned case in the absence of a reduction.

It lies within the scope of the present invention to allow the functions F1, F2, F3, F4 and the time ranges T1, T2 to consist of tables.

## Claims

1. A method of treating an air stream that is contaminated with organic substances, comprising irradiating the contaminated air stream with UV-light emitted from UV-lamps, wherein the UV-lamps have a given length of life (T1) which is determined by the decrease of the set UV-radiation in accordance with a first function (F1) from a starting state when setting the method into operation (E1) to a smallest theoretically acceptable value (E2), and wherein the UV-lamps shall be cleaned at a time interval (T2) that is determined by the reduction of the actual set radiation to a smallest desired value (E3), wherein cleaning the lamps at time intervals (T2) calculated with the aid of
- the value of the UV-radiation at the initial starting state (E1),
- the value of the smallest desired actual UV-radiation (E3),
- the first function (F1) for reduction of the UV-radiation during the working period (T).
- a second function (F2) for reducing the UV-radiation due to dirtying of the UV-lamps,
wherein the time interval (T2) is equal to the time lapsed between the point in time where the second function (F2) equates the first function (F1) and the point in time where the second function (F2) is equal to the smallest desired actual UV-radiation (E3)

2. A method according to claim 1, **characterized by** changing the second function (F2) set in the starting state and the associated time interval (T2) successively by observing the result of the treatment of the air stream and/or the dirtying of UV-lamps and/or air channels.

3. A method according to claim 1 or 2, **characterized by** adjusting the first function (F1) for the technical UV-radiation of the UV-lamps to a new first function (F11), by measuring the UV-radiation after cleaning the UV-lamps and comparing this measurement with an earlier set value for said time point, and by adjusting the second function (F2) for said UV-radiation as the lamps become dirty to a new second function (F21) by measuring the UV-radiation from the dirtied UV-lamps and comparing this radiation with the earlier set value for this time point from the earlier set second function (F2) wherein the set value of the cleaning interval (T2) is either increased or decreased (T21).

4. A method according to any one of claims 1-3, **characterized by** reducing the radiation power from the UV-lamps to a fourth function (F4) or to a desired level (E4).

5. A method according to any one of claims 1-4, **characterized by** changing the UV-light function (F2) in response to variations in the dirtying of the UV-lamps.

6. A method according to claim 4, **characterized by** changing the fourth UV-light function (F4) in response to variations in dirtying of the UV-lamps.

## Patentansprüche

1. Verfahren zum Behandeln eines Luftstroms, der mit organischen Substanzen verunreinigt ist, wobei das Verfahren das Bestrahlen des verunreinigten Luftstroms mit UV-Licht umfasst, welches von UV-Lampen emittiert wird, wobei die UV-Lampen eine gegebene Lebensdauer (T1) aufweisen, welche durch die Verringerung der eingestellten UV-Strahlung gemäß einer ersten Funktion (F1) von einem Anfangszustand, wenn das Verfahren begonnen wird (E1), auf einen geringsten theoretisch akzeptablen Wert (E2) bestimmt ist, und wobei die UV-Lampen in einem Zeitintervall (T2) gereinigt werden sollen, welches durch die Verringerung der tatsächlichen eingestellten Strahlung auf einen gewünschten geringsten Wert (E3) bestimmt ist, wobei das Reinigen der Lampen in Zeitintervallen (T2) mit Hilfe des Folgenden berechnet wird:
- des Wertes der UV-Strahlung im Startzustand (E1),
- des Wertes der gewünschten geringsten tatsächlichen UV-Strahlung (E3),
- der ersten Funktion (F1) für die Verringerung der UV-Strahlung während der Arbeitsperiode (T),
- einer zweiten Funktion (F2) für die Verringerung der UV-Strahlung aufgrund der Verschmutzung der UV-Lampen,
wobei das Zeitintervall (T2) so lang ist wie die verstrichene Zeit zwischen dem Zeitpunkt, wenn der Wert der zweiten Funktion (F2) dem Wert der ersten Funktion (F1) entspricht, und dem Zeitpunkt, wenn der Wert der zweiten Funktion (F2) der gewünschten geringsten tatsächlichen UV-Strahlung (E3) entspricht.

2. Verfahren nach Anspruch 1, **gekennzeichnet durch** das sukzessive Verändern der im Anfangszustand eingestellten zweiten Funktion (F2) und des zugehörigen Zeitintervalls (T2) nach Beobachtung des Ergebnisses der Behandlung des Luftstroms und/oder der Verschmutzung der UV-Lampen und/oder Luftkanäle.

3. Verfahren nach Anspruch 1 oder 2, **gekennzeichnet durch** das Anpassen der ersten Funktion (F1) für die technische UV-Strahlung der UV-Lampen auf eine neue erste Funktion (F11) gemäß dem Messen der UV-Strahlung nach dem Reinigen der UV-Lampen und dem Vergleich dieser Messung mit einem früher eingestellten Wert für den Zeitpunkt und **durch** das Anpassen der zweiten Funktion (F2) für die UV-Strahlung auf eine neue zweite Funktion (F21), wenn die Lampen schmutzig werden, gemäß dem Messen der UV-Strahlung von den verschmutzten UV-Lampen und dem Vergleich dieser Strahlung mit einem früher eingestellten Wert für den Zeitpunkt aus der früher eingestellten zweiten Funktion (F2), wobei der eingestellte Wert für das Reinigungsintervall (T2) entweder erhöht oder verringert wird (T21).

4. Verfahren nach einem der Ansprüche 1 bis 3, **gekennzeichnet durch** das Verringern der Strahlungsleistung der UV-Lampen auf eine vierte Funktion (F4) oder auf eine gewünschte Höhe (E4).

5. Verfahren nach einem der Ansprüche 1 bis 4, **gekennzeichnet durch** das Verändern der UV-Licht-Funktion (F2) in Reaktion auf Abweichungen bei der Verschmutzung der UV-Lampen.

6. Verfahren nach Anspruch 4, **gekennzeichnet durch** das Verändern der vierten UV-Licht-Funktion (F4) in Reaktion auf Abweichungen bei der Verschmutzung der UV-Lampen.

## Revendications

1. Méthode de traitement d'un flux d'air contaminé par des substances organiques, comprenant l'exposition du flux d'air contaminé à un rayonnement ultraviolet émis par des lampes UV, dans laquelle les lampes UV ont une durée de vie (T1) donnée qui est déterminée par la décroissance du rayonnement ultraviolet fixé suivant une première fonction (F1) depuis un état initial (E1) correspondant à la mise en marche de la méthode jusqu'à une plus petite valeur théorique acceptable (E2), et dans laquelle les lampes UV seront nettoyées à un intervalle de temps (T2) qui est déterminé par la décroissance du rayonnement fixé actuel jusqu'à une plus petite valeur voulue (E3), le nettoyage des lampes aux intervalles de temps (T2) étant calculé sur la base de
- la valeur du rayonnement ultraviolet à l'état de départ initial (E1),
- la valeur du plus petit rayonnement ultraviolet actuel voulu (E3),
- la première fonction (F1) de décroissance du rayonnement ultraviolet pendant la période de travail (T),
- une deuxième fonction (F2) de décroissance du rayonnement ultraviolet due à l'encrassement des lampes UV,
l'intervalle de temps (T2) étant égal au temps écoulé entre le point du temps où la deuxième fonction (F2) est égale à la première fonction (F1) et le point du temps où la deuxième fonction (F2) est égale au plus petit rayonnement ultraviolet actuel voulu (E3).

2. Méthode selon la revendication 1, **caractérisée en ce que** l'on fait successivement varier la deuxième fonction (F2) fixée à l'état de départ et l'intervalle de temps (T2) associé en observant le résultat du traitement du flux d'air et/ou l'encrassement des lampes UV et/ou des conduits d'air.

3. Méthode selon la revendication 1 ou la revendication 2, **caractérisée en ce que** la première fonction (F1) pour le rayonnement ultraviolet technique des lampes UV est ajustée à une nouvelle première fonction (F11) en mesurant le rayonnement ultraviolet après le nettoyage des lampes UV et en comparant cette mesure à une valeur antérieure fixée pour ledit point du temps, et **en ce que** la deuxième fonction (F2) pour ledit rayonnement ultraviolet est ajustée à mesure que les lampes s'encrassent à une nouvelle deuxième fonction (F21) en mesurant le rayonnement ultraviolet émis par les lampes UV encrassées et en comparant ce rayonnement à la valeur antérieure fixée pour ce point du temps à partir de la deuxième fonction antérieure fixée (F2), la valeur fixée pour l'intervalle de nettoyage (T2) étant soit augmentée, soit diminuée (T21).

4. Méthode selon l'une quelconque des revendications 1 à 3, **caractérisée en ce que** la puissance du rayonnement émis par les lampes UV décroît à une quatrième fonction (F4) ou à un niveau voulu (E4).

5. Méthode selon l'une quelconque des revendications 1 à 4, **caractérisée en ce que** la fonction de la lumière UV (F2) change en réponse aux variations de l'encrassement des lampes UV.

6. Méthode selon la revendication 4, **caractérisée en ce que** la quatrième fonction de la lumière UV (F4) change en réponse aux variations de l'encrassement des lampes UV.
